# EUROPÄISCHE PATENTSCHRIFT

(11) **EP 2 089 006 B1**
(45) Veröffentlichungstag und Bekanntmachung des Hinweises auf die Patenterteilung: **30.11.2011**
(21) Anmeldenummer: 07818783.8
(22) Anmeldetag: 08.10.2007
(51) Int. Cl.: A61K 9/50, A61K 9/48, A61K 31/55

(54) **GALANTHAMIN-HALTIGES ARZNEIMITTEL MIT KONTROLLIERTER FREISETZUNG**
MEDICAMENT WITH CONTROLLED RELEASE CONTAINING GALANTHAMINE
MÉDICAMENT À LIBÉRATION CONTRÔLÉE CONTENANT DE LA GALANTHAMINE

(30) Priorität: 30.11.2006 DE 102006056697
(43) Veröffentlichungstag der Anmeldung: 19.08.2009
(73) Patentinhaber: Ratiopharm GmbH, 89079 Ulm (DE)
(72) Erfinder: ALLES, Rainer, 4058 Basel (CH); MUSKULUS, Frank, 88471 Laupheim (DE); BRÜCK, Sandra, 85570 Ottenhofen (DE); SCHULZE NAHRUP, Julia, 82061 Neuried (DE)
(74) Vertreter: Best, Michael
(86) Internationale Anmeldenummer: PCT/EP2007/008709
(87) Internationale Veröffentlichungsnummer: WO 2008/064734

(56) Entgegenhaltungen:
- WO-A-00/38686
- WO-A-2005/065661

## Beschreibung

Die vorliegende Erfindung betrifft ein Arzneimittel mit kontrollierter Freisetzung. Das Arzneimittel enthält als Wirkstoff Galanthamin. Die Erfindung betrifft ebenfalls ein Verfahren zur Herstellung eines derartigen Arzneimittels und die Verwendung von Galanthamin zur Herstellung eines derartigen Arzneimittels zur Behandlung der Alzheimer-Erkrankung oder verwandter Erkrankungen.

Galanthamin ist ein tertiäres Pflanzenalkaloid, welches aus dem kleinen Schneeglöckchen (Galanthus nivalis), dem Kaukasischen Schneeglöckchen (Galanthus woronovii) sowie aus einigen Narzissenarten wie etwa der Osterglocke gewonnen werden kann. Heute kann der Wirkstoff auch auf synthetischem Wege herstellt werden.

Die chemische Bezeichnung des Galanthamins ist [4aS-(4aα,6β,8aR)]-4a,5,9,10,11,12-Hexahydro-3-methoxy-11-methyl-6H-benzofuro[3a,3,2-ef][2]benzazepin-6-ol. Galanthamin wird in Arzneimitteln in der Regel als Säureadditionssalz eingesetzt, insbesondere als Hydrobromid. Sowohl Galanthamin selbst als auch sein Hydrobromid sind linksdrehend. Galanthamin ist als Acetylcholinesteraseinhibitor, der an nikotinischen Rezeptoren, nicht jedoch an muskarinischen Rezeptoren wirkt, bekannt. Es ist weiterhin dafür bekannt, die Blut-Hirn-Schranke im Menschen zu überwinden und weist daneben bei therapeutisch wirksamen Dosen wenig ernste Nebenwirkungen auf.

Anfangs wurde Galanthamin als Wirkstoff für die Aufhebung der durch Curare-Verbindungen ausgelösten Muskelentspannungen bei Operationen eingesetzt. Heute wird Galanthamin vorwiegend als Antidementivum zur Behandlung von Demenzen, insbesondere von Alzheimer eingesetzt.

WO 97/47304 offenbart schnell auflösende Galanthamintabletten bzw. Galanthamintabletten mit sofortiger Freisetzung, die durch direktes Verpressen hergestellt werden. Diese und andere im Stand der Technik bekannten Tabletten mit sofortiger Freisetzung werden zweimal oder dreimal täglich in Abständen von 8 Stunden verabreicht.

Die Plasmaspiegel des Wirkstoffes steigen typischerweise sehr schnell an und nehmen sehr schnell wieder ab.

Die Nebenwirkungen des Galanthamins, insbesondere Übelkeit, Erbrechen, Schwitzen, Ruhelosigkeit und Schlaflosigkeit treten besonders bei Schwankungen und Sprüngen in der im Blut vorhandenen Wirkstoffkonzentration auf. Eine optimale Therapie mit Galanthamin besteht somit darin, dass die effektive Plasmakonzentration an Galanthamin möglichst konstant, d.h. tagsüber gleichmäßig hoch und nachts leicht abgesenkt konstant vorhanden bleibt.

Neuere Entwicklungen stellen daher Galanthamin-Arzneimittel mit kontrollierter, d.h. verzögerter Freisetzung des Wirkstoffs zur Verfügung, durch die über einen längeren Zeitraum eine weitgehend konstante Wirkstofffreisetzung erzielt werden soll. Derartige Galanthamin-Arzneimittel sind beispielsweise in der WO 00/38686 beschrieben. Die verzögerte Freisetzung wird durch eine die Freisetzung kontrollierende Membranbeschichtung kontrolliert, die auf der Wirkstoffschicht eines Teilchens aufgebracht ist. Auf diese Membranbeschichtung kann nochmals eine schnellfreisetzende Wirkstoffschicht aufgebracht werden, um die Freisetzung des Wirkstoffs in der ersten Stunde nach der Verabreichung zu erhöhen.

Die WO 05/065661 beschreibt Galanthamin-Arzneimittel, bei denen die Freisetzung im Wesentlichen über Acrylatpolymere eingestellt wird und die verschiedene Freisetzungsprofile aufweisen können. Bei einer Ausführungsform erfolgt eine sehr schnelle anfängliche Freisetzung von mehr als 75% des Wirkstoffs innerhalb einer halben Stunde, bei einer anderen Ausführungsform ist die Freisetzung des Galanthamins sehr langsam, so dass innerhalb der ersten Stunde weniger als 18% des Wirkstoffs freigesetzt werden.

Es besteht Bedarf nach weiteren Galanthamin-Arzneimitteln mit verzögerter Wirkstofffreisetzung, insbesondere nach Arzneimitteln, die eine sehr schnelle anfängliche Freisetzung mit einer langandauernden Freisetzung des Wirkstoffs kombinieren.

Die Aufgabe der vorliegenden Erfindung ist daher, ein Arzneimittel mit kontrollierter Freisetzung von Galanthamin zur Verfügung zu stellen, das ein bestimmtes Freisetzungsprofil mit schneller Anfangsfreisetzung und gleichmäßig über einen längeren Zeitraum anhaltender Langzeitfreisetzung aufweist. Das Freisetzungsprofil soll bevorzugt derart sein, dass bereits nach 10 Minuten 20 - 30 % des Wirkstoffs freigesetzt sind, nach 2 Stunden aber erst 30 - 45 % und nach 6 Stunden bereits über 60 % des Wirkstoffs der Formulierung freigesetzt sind.

Diese Aufgabe wird durch den Gegenstand der Patentansprüche gelöst. Die Erfindung stellt daher Arzneimittel mit kontrollierter Freisetzung zur Verfügung, umfassend ein Pellet mit einem inerten Kern, einer ersten Beschichtung auf dem inerten Kern und einer zweiten Beschichtung auf der ersten Beschichtung, dadurch gekennzeichnet, dass sowohl die erste Beschichtung als auch die zweite Beschichtung den Wirkstoff Galanthamin oder ein pharmazeutisch verträgliches Salz oder ein Solvat davon enthalten und die Zusammensetzung der ersten Beschichtung von der Zusammensetzung der zweiten Beschichtung verschieden ist. Zumindest eine der wirkstoffhaltigen Beschichtungen enthält ein wasserunlösliches filmbildendes Polymer und ist daher eine Retardierungsschicht.

Anders als bei den Galanthamin-Retard-Arzneimitteln des Standes der Technik wird bei dem erfindungsgemäßen Arzneimittel die Freisetzung nicht durch eine wirkstofffreie Membran kontrolliert, sondern die wirkstoffhaltigen Beschichtungen des Pellets selbst sind für die Retardierung des Wirkstoffs ursächlich.

Bevorzugt weisen die Pellets der erfindungsgemäßen Arzneimittel noch eine dritte Beschichtung auf, die ebenfalls den Wirkstoff Galanthamin oder ein pharmazeutisch verträgliches Salz oder Solvat davon enthält und deren Zusammensetzung von der Zusammensetzung der zweiten Beschichtung, bevorzugt sowohl von der Zusammensetzung der zweiten Beschichtung als auch von der Zusammensetzung der ersten Beschichtung, verschieden ist. In einer besonders bevorzugten Zusammensetzung weisen die erste, die zweite und die ggf. vorhandene dritte Beschichtung die gleichen Bestandteile auf, enthalten die Bestandteile aber in unterschiedlichen Mengenanteilen. Besonders bevorzugt unterscheiden sich die Beschichtungen ausschließlich in ihrem Gehalt an Wirkstoff.

Das erfindungsgemäße Arzneimittel kann noch weitere Beschichtungen aufweisen, die im Folgenden als Zwischenbeschichtung bezeichnet werden, beispielsweise zwischen dem inerten Kern und der ersten Beschichtung und/oder zwischen der ersten Beschichtung und der zweiten Beschichtung und/oder zwischen der zweiten Beschichtung und der dritten Beschichtung oder auf der dritten Beschichtung. Die Zwischenbeschichtungen unterscheiden sich von der ersten, der zweiten und der ggf. vorhandenen dritten Beschichtung dadurch, dass sie keinen Wirkstoff enthalten. Bevorzugt weisen die Pellets des erfindungsgemäßen Arzneimittels aber keine derartigen Zwischenbeschichtungen auf, d.h. die erste Beschichtung ist direkt auf dem inerten Kern angebracht, die zweite Beschichtung ist direkt auf der ersten Beschichtung angebracht und die ggf. vorhandene dritte Beschichtung ist direkt auf der zweiten Beschichtung aufgebracht. Erfindungsgemäß bevorzugt ist damit auch, dass die äußerste Beschichtung eine Beschichtung darstellt, die den Wirkstoff Galanthamin enthält. Neben den zwei notwendigen und der bevorzugten dritten wirkstoffhaltigen Beschichtung können die Pellets des erfindungsgemäßen Arzneimittels noch weitere wirkstoffhaltige Beschichtungen, d.h. eine vierte, eine fünfte und/oder eine sechste Beschichtung etc. aufweisen, bevorzugt sind aber erfindungsgemäße Pellets, die nicht mehr als vier, stärker bevorzugt Pellets, die nicht mehr als drei wirkstoffhaltige Beschichtungen aufweisen.

Als inerte Kerne für die erfindungsgemäße Formulierung kann jedes pharmazeutisch unbedenkliche inerte, vorzugsweise geschmacksneutrale Material verwendet werden. Bevorzugt sind inerte Kerne aus mikrokristalliner Cellulose. Neben mikrokristalliner Cellulose können ebenfalls Kerne aus Maisstärke oder Saccharose verwendet werden. Die Kerne sind kommerziell in verschiedenen Kornfräktionen erhältlich. Erfindungsgemäß kann jede Kerngröße verwendet werden, vorzugsweise Kerne mit Durchmessern von 100 - 1400 µm, mehr bevorzugt 500 - 1000 µm, insbesondere 650 - 750 µm, z.B. etwa 700 µm. Mikrokristalline Cellulose ist besonders aufgrund fehlender Wasserlöslichkeit, geringer Friabilität (=Abrieb) und konstanter Rundheit bevorzugt. Des Weiteren werden durch den inerten Charakter der mikrokristallinen Cellulose unerwünschte Wechselwirkungen mit dem Wirkstoff vermieden.

Als inerte Kerne in den Zusammensetzungen der vorliegenden Erfindung können neben den oben bevorzugt genannten weitere verschiedene Materialien eingesetzt werden, vorausgesetzt diese Materialien sind pharmazeutisch unbedenklich und weisen geeignete Dimensionen und Festigkeiten auf. Beispiele solcher Materialien sind Polymere, z.B. Kunststoffharze, anorganische Substanzen, z.B. Siliziumdioxid, Glas, Hydroxylapatit, Salze (Natrium- oder Kaliumchlorid, Calcium- oder Magnesiumcarbonat) und der gleichen, organische Substanzen, z.B. Aktivkohle, Säuren (Zitronensäure, Fumarsäure, Weinsäure, Ascorbinsäure und ähnliche Säuren) und Saccharide und deren Derivate. Besonders geeignete Materialien sind Saccharide wie Zucker, Oligosaccharide, Polysaccharide und deren Derivate, beispielsweise Glukose, Rhamnose, Galaktose, Laktose, Saccharose, Mannit, Sorbit, Dextrin, Maltodextrin, Cellulose oder deren Derivate, Stärken (Mais, Reis, Kartoffel, Weizen, Tapioca) und ähnliche Saccharide.

Zumindest eine der erfindungsgemäßen wirkstoffhaltigen Beschichtungen weist ein wasserunlösliches filmbildendes Polymer auf und ist daher eine Retardschicht. Erfindungsgemäß bevorzugt weisen alle wirkstoffhaltigen Beschichtungen ein wasserunlösliches filmbildendes Polymer auf und bevorzugt setzen daher alle wirkstoffhaltigen Beschichtungen den Wirkstoff retardiert frei. Derartige wasserunlösliche filmbildende Polymere sind zur Herstellung von Retard-Überzügen bekannt.

Die wasserunlöslichen, filmbildenden Polymere können z.B. ein Celluloseether wie Ethylcellulose, ein Celluloseester wie Celluloseacetat oder ein Polyvinylalkohol sein. Besonders bevorzugt ist Ethylcellulose. Neben dem wasserunlöslichen Polymer, wie Ethylcellulose, können die Beschichtungen ggf. weitere übliche Bestandteile von Retardbeschichtungen enthalten, z.B. ein weiteres wasserlösliches Polymer zur Einstellung der Freisetzung und/oder einen Weichmacher. Ein geeignetes wasserlösliches Polymer ist z.B. Hydroxypropylmethylcellulose. Als Weichmacher in den Beschichtungsformulierungen können alle gängigen, pharmazeutisch unbedenklichen Weichmacher für wasserunlösliche Polymere, wie Ethylcellulose, verwendet werden. Geeignet sind z.B. Triethylcitrat oder Polyethylenglykol, am meisten bevorzugt ist Triethylcitrat. Der Weichmacher wird in den Dispersionen zum Beschichten der inerten Kerne in einer Menge von vorzugsweise 0,2 - 5 Gew.-%, insbesondere von 0,5 - 4,5 Gew.-% eingesetzt, jeweils bezogen auf das Gesamtgewicht der wässrigen Dispersion. Durch das wasserlösliche Polymer wird gewöhnlich die Freisetzung des Wirkstoffs beschleunigt. Bevorzugt wird jedoch kein wasserlösliches Polymer in den wirkstoffhaltigen Beschichtungen der Pellets der Formulierungen der vorliegenden Erfindung eingesetzt.

Neben den oben genannten Inhaltsstoffen können die erfindungsgemäßen Beschichtungen noch verschiedene übliche Zusatzstoffe wie Verdickungsmittel, Schmiermittel, Tenside, Konservierungsstoffe, Komplexbildner, Chelatbildner und/oder Elektrolyte enthalten.

Die relativen Anteile der Bestandteile der Beschichtungen, insbesondere das Verhältnis von wasserunlöslichem, filmbildendem Polymer zu den anderen Bestandteilen, bestimmen das Freisetzungsprofil der Beschichtung.

Das Beschichtungsmaterial muss geeignet sein, mit dem Wirkstoff vermischt zu werden, und diesen verzögert freizusetzen. Als besonders bevorzugt hat sich eine Mischung aus Ethylcellulose, einem Tensid wie Natriumlaurylsulfat und Cetylalkohol (z.B. eine solche Mischung, wie sie als AQUACOAT^{™} kommerziell erhältlich ist) mit Triethylcitrat als Weichmacher als Beschichtungsmaterial herausgestellt.

Die inerten Kerne werden zur Herstellung der erfindungsgemäßen Formulierung vorzugsweise mit wässrigen Dispersionen der Beschichtungsmaterialien, in die der Wirkstoff eingemischt wird, beschichtet.

Die vorstehend beschriebenen Beschichtungen enthalten neben dem wasserunlöslichen Polymer und den ggf. weiteren Bestandteilen noch den Wirkstoff des Arzneimittels. Der Wirkstoff ist erfindungsgemäß Galanthamin, das als freie Base, bevorzugt aber in Form eines pharmazeutisch verträglichen Salzes, insbesondere in Form eines pharmazeutisch verträglichen Säureadditionssalzes vorliegt. Der Wirkstoff bzw. das Salz kann auch als Solvat, z.B. Hydrat, vorliegen. Wenn im Rahmen dieser Anmeldung von dem "Wirkstoff" gesprochen wird, ist hierunter der Wirkstoff in Form der freien Basen oder in Form eines pharmazeutisch verträglichen Salzes, insbesondere eines pharmazeutisch verträglichen Säureadditionssalzes zu verstehen, wobei die freie Base oder das Salz auch in Form eines Solvates, insbesondere eines Hydrates vorliegen kann. Besonders bevorzugt liegt der Wirkstoff erfindungsgemäß als Galanthaminhydrobromid vor.

Neben dem Wirkstoff Galanthamin (bzw. dem Salz oder Solvat davon) kann das erfindungsgemäße Arzneimittel noch von Galanthamin verschiedene Wirkstoffe enthalten, bevorzugt enthält das erfindungsgemäße Arzneimittel aber ausschließlich den Wirkstoff Galanthamin (bzw. ein Salz oder Solvat davon) und keinen weiteren Wirkstoff.

Neben den vorstehend beschriebenen erfindungsgemäßen Beschichtungen, die den Wirkstoff enthalten, kann das erfindungsgemäße Pellet noch weitere Beschichtungen aufweisen, die keinen Wirkstoff enthalten und die im Rahmen dieser Anmeldung als "Zwischenbeschichtungen" bezeichnet werden. Die Zwischenbeschichtungen können ebenfalls retardierende, d.h. die Freisetzung verzögernde Beschichtungen darstellen oder einfache Inert-Trennbeschichtungen sein, die die erfindungsgemäßen wirkstoffhaltigen Beschichtungen voneinander trennen und die in der Regel aus einem Bindemittel wie Hydroxypropylmethylcellulose und anderen üblichen Zusatzstoffen bestehen. Bevorzugt weist das erfindungsgemäße Pellet aber keine derartigen Zwischenbeschichtungen auf, sondern besteht aus dem inerten Kern und den darauf aufgebrachten wirkstoffhaltigen Beschichtungen.

In einer bevorzugten Ausführungsform der vorliegenden Erfindung enthalten die verschiedenen Beschichtungen unterschiedliche Mengen von Wirkstoff. Mehr bevorzugt enthalten die weiter innen liegenden Schichten höhere Konzentrationen von Wirkstoff als die weiter außen liegenden Schichten. Besonders bevorzugt weisen die Pellets der erfindungsgemäßen Formulierung drei Schichten mit jeweils unterschiedlichen Wirkstoffkonzentrationen auf, wobei die innerste Schicht die höchste Wirkstoffkonzentration und die mittlere Schicht die geringste Wirkstoffkonzentration aufweist. So liegt bei einem erfindungsgemäßen Pellets mit zwei Beschichtungen die Wirkstoffkonzentration in der inneren Schicht vorzugsweise bei 5 - 10 Gew.-%, insbesondere 6 - 8 Gew.-%, und in der äußeren Schicht bevorzugt bei 0,5 - 5 Gew.-%, insbesondere 1 - 3 Gew.-%. Bei der besonders bevorzugten Ausführungsform mit drei Beschichtungen liegt die Wirkstoffkonzentration in der innersten Schicht vorzugsweise bei 4 - 8 Gew.-%, insbesondere bei 5 - 7 Gew.-%, z.B. bei etwa 6 Gew.-%, in der mittleren Schicht bei 0,2 bis 2 Gew.-%, insbesondere bei 0,3 - 1 Gew.-%, z.B. bei etwa 0,5 Gew.-%, und in der äußeren Schicht bei bevorzugt 0,5 - 5 Gew.-%, insbesondere 1 - 3 Gew.-%, z.B. etwa 1,5 Gew.-%. Die vorstehenden Prozentangaben beziehen sich jeweils auf das Gesamtgewicht eines Pellets. Gleiches gilt generell für Prozentangaben im Rahmen der vorliegenden Erfindung, sofern nichts anderes ausdrücklich angegeben oder für einen Fachmann offensichtlich ist.

Die Pellets des Arzneimittels der vorliegenden Erfindung können auf verschiedene im Stand der Technik bekannte Weise in eine therapeutisch wirksame Dosierungseinheit überführt werden. Z.B. können die Pellets in eine Hartgelatinekapsel abgefüllt werden, sodass eine therapeutisch wirksame Menge von vorzugsweise 5 - 40 mg, insbesondere 8 - 32 mg Wirkstoff pro Dosierungseinheit zur Verfügung gestellt wird. Neben Hartgelatinekapseln können die inerten Partikel nach im Stand der Technik bekannten Verfahren ebenfalls in Weichgelatinekapseln abgefüllt werden oder zu Tabletten verpresst oder in Beutel abgefüllt werden, wobei eine Dosierungseinheit bevorzugt ist, die die vorstehend angegebenen Mengen an Wirkstoff aufweist. Vorzugsweise werden die inerten Partikel in Hartgelatinekapseln abgefüllt.

Erfindungsgemäß bevorzugt handelt es sich daher bei den Arzneimitteln, die ein oder bevorzugt mehrere Pellets umfassen, um Hartgelatinekapseln, Tabletten oder Beutel, besonders bevorzugt um Hartgelatinekapseln.

In einer besonders bevorzugten Ausführungsform der vorliegenden Erfindung weist die erfindungsgemäße Formulierung ein Freisetzungsprofil des Wirkstoffes auf, worin nach 10 Minuten 15 - 35 %, vorzugsweise 20 - 30 %, nach 2 Stunden 25 - 50 %, vorzugsweise 30 - 45 %, insbesondere 35 - 45 %, und nach 6 Stunden über 55, vorzugsweise über 60, insbesondere über 70 % des Wirkstoffs freigesetzt werden. Besonders bevorzugt sind Formulierungen, die ein Freisetzungsprofil aufweisen, worin nach 2 Stunden 35 - 45 % des Wirkstoffs freigesetzt werden und solche Formulierungen, die ein Freisetzungsprofil aufweisen, worin nach 6 Stunden über 70 % des Wirkstoffs freigesetzt werden.

Unter "Freisetzungsprofil" wird im Rahmen der vorliegenden Erfindung der zeitliche Verlauf der Menge des Wirkstoffs, die gelöst vorliegt, bezogen auf die Gesamtmenge des Wirkstoffs verstanden. Dieses Freisetzungsprofil wird erhalten, indem die Pellets der vorliegenden Erfindung in einem USP-Aparat II (Paddle-Apparatur) in 900 ml USP-Puffer (Phosphatpuffer) bei pH 6,8, 37°C und einer Rührgeschwindigkeit von 75 U/min gelöst und die Menge des gelösten Wirkstoffs über einen Zeitraum, z.B. 24 Stünden, zu verschiedenen Zeitpunkten, z.B. alle 5 Minuten, gemessen wird. Der Gehalt an freigesetztem Wirkstoff kann auf bekannte Art und Weise, z.B. durch Absorption des Lichts einer bestimmten Wellenlänge oder über HPLC oder ein anderes geeignetes Nachweisverfahren erfolgen. Im Übrigen entsprechen die Versuchsbedingungen für die Bestimmung des Freisetzungsprofils den Vorschriften der US-Pharmacopoeia.

Die vorliegende Erfindung betrifft weiterhin Verfahren zur Herstellung von oben beschriebenen Formulierungen, bei denen man einen inerten Kern mit mindestens zwei Beschichtungen versieht, wobei die Beschichtungen Galanthamin oder ein pharmazeutisch verträgliches Salz oder ein Solvat davon enthalten.

Die Pellets des Arzneimittels der vorliegenden Erfindung lassen sich auf übliche Art und Weise herstellen. Die Beschichtung kann auf wässrige oder nicht-wässrige Art und Weise erfolgen. Bei einer wässrigen Verfahrensführung, die erfindungsgemäß bevorzugt ist, wird z.B. eine entsprechende Menge an Galanthamin oder ein pharmazeutisch verträgliches Salz oder Solvat davon in einer geeigneten Menge Wasser aufgelöst oder dispergiert. Hierzu werden die weiteren Bestandteile der Beschichtung, z.B. der ggf. vorhandene Weichmacher zugesetzt. Das wasserunlösliche Polymer wird dann bevorzugt als wässrige Dispersion, die in der Regel noch zumindest ein Tensid enthält, mit der Lösung bzw. der Dispersion des Wirkstoffs vermischt. Die inerten Kerne werden mit dieser Dispersion beschichtet. Für die weiteren Beschichtungen werden analog weitere Dispersionen mit entsprechenden Zusammensetzungen bereitet und aufgebracht. Für eine nicht-wässrige Beschichtungsform können übliche Lösemittel wie Alkohole, z.B. Ethanol, eingesetzt werden. Die Menge an Wirkstoff in der das Arzneimittel enthaltenden Beschichtungslösung / Dispersion kann im Bereich von 0,1 - 10 Gew.-% liegen, vorzugsweise 1 - 5 Gew.-%, insbesondere 2 - 3 Gew.-%, jeweils bezogen auf das Gesamtgewicht der Dispersion bzw. Lösung.

Das Beschichten der inerten Kerne kann z.B. in einem Wirbelschichtgranulator (z.B. Glatt Typ BSG-30 oder GPCG-30) durchgeführt werden, der vorzugsweise mit einem Wurster-Bottom-Spray-Einsatz (z.B. einem 18-Zoll-Wurster-Einsatz) ausgestattet ist. Die Verfahrensparameter hängen von dem verwendeten Gerät ab und werden von einem Fachmann auf übliche Art und Weise eingestellt.

Die Sprühgeschwindigkeit während der Beschichtung sollte sorgfältig eingestellt werden. Eine zu niedrige Sprühgeschwindigkeit kann dazu führen, dass ein Teil der Beschichtungslösung/-dispersion, die das Arzneimittel enthält, sprühgetrocknet wird, und es zu Materialverlust kommt. Eine zu hohe Sprühgeschwindigkeit kann zu einer zu starken Benetzung der Partikel mit anschließender Agglomeration von Material führen. Vorzugsweise wird mit niedrigerer Sprühgeschwindigkeit begonnen und die Sprühgeschwindigkeit wird während des Beschichtung langsam erhöht.

Die Pellets können in automatischen Standart-Kapselfüllmaschinen in Hartgelatinekapseln abgefüllt werden. Die Herstellung von Darreichungsformen wie Weichgelatinekapsel, Beutel, Tablette, oder anderen sind im Stand der Technik bekannt.

Des Weiteren betrifft die vorliegende Erfindung die Verwendung einer therapeutisch wirksamen Menge von Galanthamin in einem wie oben beschriebenen Arzneimittel mit kontrollierter Freisetzung zur Herstellung eines Medikaments zur Behandlung von Alzheimer-Krankheit und verwandten Krankheiten. Hierbei werden insbesondere die in Verbindung mit Acetylcholesterinesteraseinhibitoren auftretenden Nebenwirkungen reduziert, ohne jedoch die Menge des Wirkstoffes reduzieren zu müssen. Weitere verwandte Krankheiten, die mit den Formulierungen der vorliegenden Erfindung behandelt werden können, sind z.B. vaskuläre Demenz, Lewy-Korpus-Krankheit, Autismus, mentale Retardierung, bipolare psychiatrische Erkrankungen, gestörtes Verhalten, Aufmerksamkeits-Defizit-Hyperaktivitäts-Syndrom (ADHS), Substanzmissbrauch, extreme Aggression, Verhaltensstörungen, Absetzen von Nikotin und Nikotinentzug.

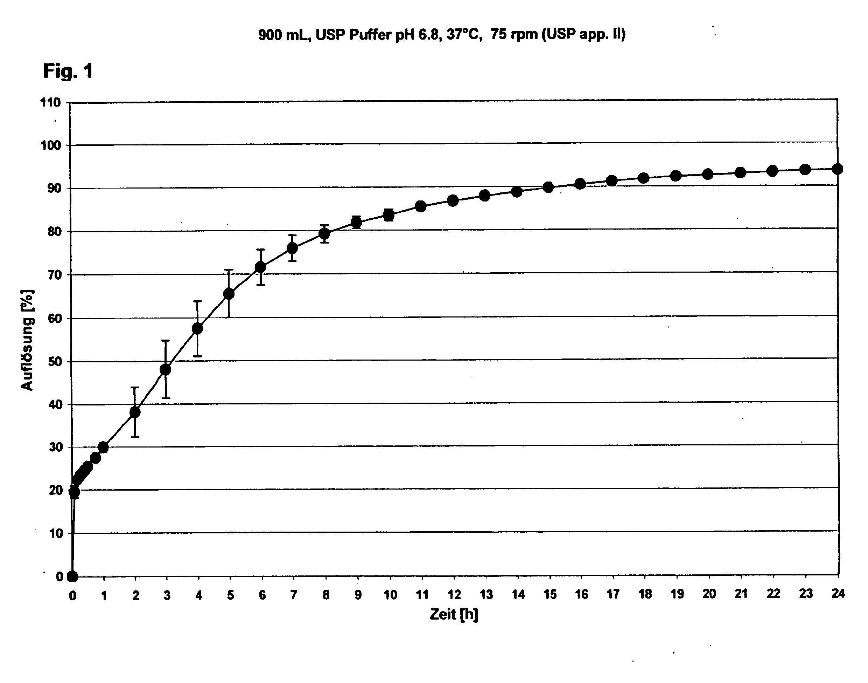

Figur 1 beschreibt ein Auflösungsprofil der erfindungsgemäßen Pellets (900 mL, USP-Puffer, pH 6,8, 37°C, 75 rpm, USP app. II)

Die Erfindung wird durch das folgende Beispiel weiter veranschaulicht:

### Beispiel 1

Erfindungsgemäße Pellets wurden aus folgenden Bestandteilen hergestellt:

| Nr. | Zusammensetzung | |
|---|---|---|
| | **Vorlage** | mg |
| 1 | Cellets 700 (mikrokristalline Cellulose) | 110,00 |
| | **Dispersion I** | |
| 2 | Galanthamin HBr | 12,00 |
| 3 | Wasser, gereinigt | 531,00 |
| 4 | TEC | 10,00 |
| 5 | Aquacoat ECD | 50,00 |
| | **Dispersion II** | |
| 6 | Galanthamin HBr | 1,00 |
| 7 | Wasser, gereinigt | 35,40 |
| 8 | TEC | 2,00 |
| 9 | Aquacoat ECD | 10,00 |
| | **Dispersion III** | |
| 10 | Galanthamin HBr | 3,00 |
| 11 | Wasser, gereinigt | 106,13 |
| 12 | TEC | 0,60 |
| 13 | Aquacoat ECD | 3,00 |
| | GESAMTMENGE **Feststoffe**: | 201,60 |

Die Bestandteile wurden wie folgt verarbeitet:
Dispersion I wurde hergestellt, indem das Galanthaminsalz in Wasser gelöst wurde, TEC wurde mit Aquacoat vermischt und in die Galanthamin-Lösung eingerührt. Die Kerne Cellets 700 wurden im Wirbelschichtgerät beschichtet. Die so hergestellten Pellets wurden getrocknet. Anschließend wurden die Dispersionen II und III entsprechend hergestellt und aufgetragen.

Die in Beispiel 1 erhaltenen Pellets wurden einem Freisetzungstest unterzogen, und das Freisetzungsprofil wurde wie vorstehend angegeben bestimmt.

Das Ergebnis des Versuchs ist in Figur 1 gezeigt. Die erfindungsgemäßen Pellets weisen insbesondere eine schnelle Anfangsfreisetzung bei konstant langanhaltend guter Langzeitfreisetzung auf.

## Patentansprüche

1. Arzneimittel mit kontrollierter Freisetzung, umfassend ein Pellet mit einem inerten Kern, einer ersten Beschichtung auf dem inerten Kern und einer zweiten Beschichtung auf der ersten Beschichtung, **dadurch gekennzeichnet, dass** sowohl die erste Beschichtung als auch die zweite Beschichtung den Wirkstoff Galanthamin oder ein pharmazeutisch verträgliches Salz oder Solvat davon enthalten, die Zusammensetzung der ersten Beschichtung von der Zusammensetzung der zweiten Beschichtung verschieden ist und zumindest eine der Beschichtungen ein filmbildendes wasserunlösliches Polymer enthält.

2. Arzneimittel nach Anspruch 1, bei dem das Pellet auf der zweiten Beschichtung eine dritte Beschichtung aufweist, die den Wirkstoff Galanthamin oder ein pharmazeutisch verträgliches Salz oder Solvat davon enthält und deren Zusammensetzung von der Zusammensetzung der zweiten Beschichtung verschieden ist.

3. Arzneimittel nach Anspruch 2, bei dem die Zusammensetzung der ersten Beschichtung von der Zusammensetzung der dritten Beschichtung verschieden ist.

4. Arzneimittel nach einem der vorstehenden Ansprüche, wobei die Beschichtungen sich in ihrem Gehalt an Wirkstoff unterscheiden.

5. Arzneimittel nach einem der vorstehenden Ansprüche, bei dem die zweite Beschichtung des Pellets direkt auf der ersten Beschichtung aufgebracht ist und die gegebenenfalls vorhandene dritte Beschichtung direkt auf der zweiten Beschichtung aufgebracht ist.

6. Arzneimittel nach einem der vorstehenden Ansprüche, bei dem die äußerste Beschichtung des Pellets die zweite Beschichtung bzw. die gegebenenfalls vorhandene dritte Beschichtung ist.

7. Arzneimittel nach einem der vorstehenden Ansprüche, bei dem das Pellet keine die Freisetzung kontrollierende Beschichtung aufweist, die wirkstofffrei ist.

8. Arzneimittel nach einem der vorstehenden Ansprüche, bei dem alle wirkstoffhaltigen Beschichtungen des Pellets ein filmbildendes, wasserunlösliches Polymer aufweisen.

9. Arzneimittel nach einem der vorstehenden Ansprüche, wobei der Wirkstoff Galanthaminhydrobromid ist.

10. Arzneimittel nach einem der vorstehenden Ansprüche, das folgende nach der Paddle-Methode der USP (900 ml, wässrig, USP-Puffer pH 6,8, 37°C, USP app. II) bestimmte Freisetzung des Wirkstoffs aufweist:
20 - 30% nach 10 Minuten
30 - 45 % nach 2 Stunden
mehr als 60% nach 6 Stunden.

11. Arzneimittel nach einem der vorstehenden Ansprüche in Form einer Hartgelatinekapsel, die Pellets enthält, eines Beutels, der Pellets enthält oder einer aus Pellets verpressten Tablette.

12. Verwendung von Galanthamin oder eines pharmazeutisch verträglichen Salzes oder Solvates davon zur Herstellung eines Arzneimittels wie in einem der vorstehenden Ansprüche definiert zur Behandlung der Alzheimer-Krankheit oder einer verwandten Krankheit beim Menschen.

13. Verwendung nach Anspruch 12, wobei die verwandte Krankheit ausgewählt ist aus der Gruppe, bestehend aus vaskulärer Demenz, Lewy-Korpus-Krankheit, Autismus, mentaler Retardierung, bipolaren psychiatrischen Erkrankungen, Aufmerksamkeits-Defizit-Hyperaktivitäts-Syndrom (ADHS), Substanzmissbrauch, extreme Aggression, Verhaltensstörungen und Nikotinentzugserscheinungen.

## Claims

1. A medicament having controlled release, comprising a pellet having an inert core, a first coating on the inert core and a second coating on the first coating, **characterized in that** both the first coating and the second coating contain the active substance galanthamine or a pharmaceutically acceptable salt or solvate thereof, the composition of the first coating differs from the composition of the second coating and at least one of the coatings contains a film-forming, water insoluble polymer.

2. The medicament according to claim 1, wherein the pellet has on the second coating a third coating which contains the active substance galanthamine or a pharmaceutically acceptable salt or solvate thereof and whose composition differs from the composition of the second coating.

3. The medicament according to claim 2, wherein the composition of the first coating differs from the composition of the third coating.

4. The medicament according to any of the preceding claims, wherein the coatings differ as regards their active substance content.

5. The medicament according to any of the preceding claims, wherein the second coating of the pellet is directly applied to the first coating and the optionally present third coating is directly applied to the second coating.

6. The medicament according to any of the preceding claims, wherein the outermost coating of the pellet is the second coating or the optionally present third coating.

7. The medicament according to any of the preceding claims, wherein the pellet has no release controlling coating which is free of active substance.

8. The medicament according to any of the preceding claims, wherein all active substance containing coatings of the pellet have a film-forming, water insoluble polymer.

9. The medicament according to any of the preceding claims, wherein the active substance is galanthamine hydrobromide.

10. The medicament according to any of the preceding claims, which has the following active substance release determined according to the Paddle method of USP (900 ml, aqueous, USP buffer, pH 6.8, 37°C, USP app. II):
20 - 30 % after 10 minutes
30 - 45 % after 2 hours
over 60 % after 6 hours.

11. The medicament according to any of the preceding claims in the form of a hard gelatin capsule containing pellets, a sachet containing pellets or a tablet compressed from pellets.

12. Use of galanthamine or a pharmaceutically acceptable salt or solvate thereof for the production of a medicament as defined in any of the preceding claims for the treatment of Alzheimer's disease or a related disease in man.

13. Use according to claim 12, wherein the related disease is selected from the group consisting of vascular dementia, Lewy body disease, autism, mental retardation, bipolar psychiatric diseases, attention deficit hyperactivity syndrome (ADHS), substance abuse, extreme aggression, disordered behaviors, and nicotine withdrawal syndromes.

## Revendications

1. Médicament à libération contrôlée, comprenant une pastille avec un noyau inerte, un premier revêtement sur le noyau inerte et un deuxième revêtement sur le premier revêtement, **caractérisé en ce que** tant le premier revêtement que le deuxième revêtement contiennent l'agent actif galantamine ou un sel ou solvate de cet agent compatible au plan pharmaceutique, la composition du premier revêtement étant différente de la composition du deuxième revêtement et au moins l'un des revêtements contenant un polymère filmogène et insoluble dans l'eau.

2. Médicament selon la revendication 1, dans lequel la pastille présente sur le deuxième revêtement un troisième revêtement qui contient l'agent actif galantamine ou un sel ou solvate de cet agent compatible au plan pharmaceutique et dont la composition est différente de la composition du deuxième revêtement.

3. Médicament selon la revendication 2, dans lequel la composition du premier revêtement est différente de la composition du troisième revêtement.

4. Médicament selon l'une des revendications précédentes, dans lequel les revêtements diffèrent par leur teneur en agent actif.

5. Médicament selon l'une des revendications précédentes, dans lequel le deuxième revêtement de la pastille est appliqué directement sur le premier revêtement et le troisième revêtement éventuellement présent est appliqué directement sur le deuxième revêtement.

6. Médicament selon l'une des revendications précédentes, sur lequel le revêtement le plus extérieur de la pastille est le deuxième revêtement ou le troisième revêtement éventuellement présent.

7. Médicament selon l'une des revendications précédentes, dans lequel la pastille ne présente pas de revêtement qui contrôle la libération et est exempt d'agent actif.

8. Médicament selon l'une des revendications précédentes, dans lequel tous les revêtements de la pastille contenant un agent actif présentent un polymère filmogène et insoluble dans l'eau.

9. Médicament selon l'une des revendications précédentes, dans lequel l'agent actif est du bromhydrate de galantamine.

10. Médicament selon l'une des revendications précédentes, présentant la libération suivante de l'agent actif laquelle est déterminée selon la méthode de Paddle d'après USP (900 ml de tampon USP, aqueux, pH 6,8, 37°C, USP app. II) :
20 - 30 % après 10 minutes
30 - 45 % après 2 heures
plus de 60 % après 6 heures.

11. Médicament selon l'une des revendications précédentes sous la forme d'une capsule de gélatine dure qui contient des pastilles, d'un sachet qui contient des pastilles ou d'un comprimé compacté à partir de pastilles.

12. Utilisation de galantamine ou d'un sel ou d'un solvate de cet agent compatible au plan pharmaceutique pour la fabrication d'un médicament comme défini dans l'une des revendications ci-dessus pour le traitement de la maladie d'Alzheimer ou d'une maladie apparentée chez l'homme.

13. Utilisation selon la revendication 12, la maladie apparentée étant choisie dans le groupe comprenant la démence vasculaire, la maladie à corps de Lewy, l'autisme, le retard mental, des maladies psychiatriques bipolaires, un syndrome d'hyperactivité avec déficit d'attention (ADHS), un abus de substances, une agressivité extrême, des troubles du comportement et des symptômes de sevrage de la nicotine.
